# EUROPEAN PATENT APPLICATION

(11) **EP 2 133 106 A1**
(43) Date of publication of application: **16.12.2009**
(21) Application number: 08720339.4
(22) Date of filing: 05.03.2008
(51) Int. Cl.: A61M 1/12

(54) **AUXILIARY ARTIFICIAL HEART APPARATUS**

(30) Priority: 05.03.2007 JP 2007054029
(71) Applicant: JMS Co., Ltd., Hiroshima-shi, Hiroshima 730-8652 (JP)
(72) Inventor: MATSUI, Yoshiro, Sapporo-shi Hokkaido 064-0823 (JP); HAYASHI, Shuro, Hiroshima-shi Hiroshima 730-8652 (JP); TANAKA, Takeshi, Aki-gun Hiroshima 736-0032 (JP)
(74) Representative: Collin, Jérôme
(86) International application number: PCT/JP2008/000453
(87) International publication number: WO 2008/108100

(57) **Abstract**

Heart side magnets 20 and 21 are fixedly secured to the surface of a heart 100. A material which has a higher rigidity than the cardiac muscle is molded to form a molded member 30. Molded member side magnets 40 and 41 are attached to the molded member 30 so as to correspond to the heart side magnets 20 and 21. The polarity of one of the heart side magnets 20 and 21 and the molded member side magnets 40 and 41 is switched at predetermined timings, whereby the heart 100 is magnetically expanded and contracted to improve the pumping function.

## Description

### Technical Field

The present invention relates to heart assist devices for use in heart disease patients.

### Background Art

Conventionally, for example, heart disease patients in whom the pumping function of the heart significantly deteriorates, such as dilated cardiomyopathy patients, are subjected to the operation of replacing the heart of the patient with an artificial heart or the operation of implanting an artificial heart for assisting the pumping function of the heart. A known artificial heart of this type is one that includes a mechanical pump with a rotor, such as an impeller, or the like, as disclosed in Patent Document 1, for example. In the artificial heart of Patent Document 1, the blood is once sucked into a pump housing by rotation of the impeller and then discharged into the body of the patient.

Patent Document 1: Japanese Laid-Open Patent Publication No. 2003-24434

### Disclosure of Invention

### Problems to be Solved by the Invention

However, when the blood is sucked into a mechanical pump and discharged as in the artificial heart of Patent Document 1, the blood is once extracted out of a blood vessel and then returned to the blood vessel. Therefore, there is a probability that air enters the blood in midstream. Also, the blood flow which is generally produced by pulsation of the heart is difficult to produce only by spinning the impeller.

When the blood is discharged by the mechanical pump of Patent Document 1, the shear force generated by the rotation of the impeller acts on erythrocytes in the blood. The erythrocytes subjected to the shear force readily collapse because they do not have cytoskeleton which could maintain the cell shape in the cell membrane. When the erythrocytes collapse, it is possible that cell contents come out and condensation of thrombocytes occurs, resulting in formation of a thrombus. When the mechanical pump is used, the blood comes in direct contact with an artificial object. This can be another cause of formation of a thrombus.

To prevent formation of the above-described thrombus, the patient needs to continue to take medication. This is a large burden on the patient. It is also possible that formation of a thrombus occurs even in a patient who takes medication because of, for example, the predisposition of the patient.

In the heart failure patients, diastolic failure of the heart frequently occurs as well as systolic failure. In the context of assisting the pumping function of the heart, assisting the expansion is also important. It is also possible that an asynchronous movement occurs at the site of the myocardial wall due to an intraventricular conduction disturbance or the like. In the case of such a symptom exhibited, a heart resynchronization therapy is carried out by biventricular pacing, although the effects of this therapy on seriously diseased patients are not so good as expected.

The present invention was conceived in view of the above circumstances. An object of the present invention is to provide a heart assist device capable of improving not only the diastolic pumping function but also the systolic pumping function performed based on pulsation of the patient's own heart, while entrance of air into the blood and formation of a thrombus due to collapse of erythrocytes are prevented so that the burden on the patient is reduced, and capable of controlling the asynchronous movement of the myocardial wall to restore normal operation.

### Means for Solving the Problems

To achieve the above object, the present invention magnetically applies a contraction force and an expansion force to the heart by switching the polarity of one of a magnetic force generator fixedly secured to the heart and another magnetic force generator provided to a molded member which is molded so as to cover the heart at predetermined timings.

Specifically, the first invention is configured to include a heart side magnetic force generator fixedly secured to a surface of a heart; a molded member formed of a material having a higher rigidity than a cardiac muscle so as to cover the heart; a molded member side magnetic force generator provided to the molded member so as to correspond to the heart side magnetic force generator; and a polarity switching unit for switching a polarity of one of the heart side magnetic force generator and the molded member side magnetic force generator at predetermined timings.

In this structure, for example, when the polarity of the molded member side magnetic force generator is changed by the polarity switching unit so as to be equal to that of the heart side magnetic force generator, a repulsive force is generated between the molded member side magnetic force generator and the heart side magnetic force generator. Here, the heart side magnetic force generator moves in a direction away from the molded member side magnetic force generator because the molded member is formed of a material having a higher rigidity than the cardiac muscle so as to cover the heart so that the molded member side magnetic force generator hardly moves relative to the heart. Accordingly, the heart side magnetic force generator is pressed against the heart. The heart against which the heart side magnetic force generator is pressed contracts. When in this state the polarity of the molded member side magnetic force generator is changed by the polarity switching unit so as to be different from that of the heart side magnetic force generator, the heart side magnetic force generator is attracted by the molded member side magnetic force generator. Here, the heart expands because the heart side magnetic force generator is fixedly secured to the heart. Specifically, a contraction force and an expansion force can be repeatedly applied to the heart of the patient by switching the polarity of the molded member side magnetic force generator using the polarity switching unit. Thus, the heart of the patient can be caused to pulsate. Note that a contraction force and an expansion force can also be applied to the heart by switching the polarity of the heart side magnetic force generator using the polarity switching unit.

Also, an expanded dysfunctional heart can be contracted by an external compressive force and expanded by an external attractive force. Entrance of air into the blood and direct contact of the blood with an artificial object can be avoided. Therefore, formation of a thrombus can be prevented. Also, assistance can be given not only in the case of systolic failure but also in the case of diastolic failure which frequently occurs in heart failure patients. Further, the asynchronous movement of the myocardial wall due to, for example, an intraventricular conduction disturbance can be controlled to restore normal operation.

According to the second invention, in the first invention, a cover member is further provided for covering the surface of the heart, wherein the heart side magnetic force generator is attached to the cover member.

In this structure, the heart side magnetic force generator can be fixedly secured to the heart without substantially damaging the cardiac muscle.

According to the third invention, in the first invention, the heart side magnetic force generator includes a plurality of magnetic force generators.

In this structure, a contraction force and an expansion force generated by a magnetic force can be dispersedly exerted without being locally exerted on part of the heart.

According to the fourth invention, in the third invention, the heart side magnetic force generators are fixedly secured to a front surface and a rear surface of the heart.

In this structure, the contraction force and the expansion force can be exerted on the heart on both the front and rear sides.

According to the fifth invention, in the first invention, the molded member is composed of a front divisional part and a rear divisional part which are separate with respect to an anteroposterior direction of the heart, and the front divisional part and the rear divisional part each have a binding portion at which the front divisional part and the rear divisional part are joined together.

In this structure, the molded member can be placed so as to cover the heart only by placing the heart between the front divisional part and the rear divisional part with the divisional parts being separate from each other and joining the front divisional part and the rear divisional part together at the binding portions.

According to the sixth invention, in the first invention, the molded member has a space into which the heart is to be inserted with a heart apex foremost.

In this structure, the molded member can be placed so as to cover the heart only by inserting the heart into the space with the heart apex foremost.

### Effects of the Invention

According to the first invention, the polarity of one of the heart side magnetic force generator fixedly secured to the heart and the molded member side magnetic force generator provided to the molded member which covers the heart is switched by a polarity switching unit at predetermined timings. Therefore, a contraction force and an expansion force generated by a magnetic force can be exerted on the patient's own heart to pulsate without using a conventional mechanical pump, and both the systolic pumping function and the diastolic pumping function can be improved, while the asynchronous movement of the myocardial wall can be controlled to restore normal operation. Since the patient's own heart can be caused to pulsate in this way, entrance of air into the blood is avoided, and formation of a thrombus rarely occurs. Thus, the burden on the patient can be reduced.

According to the second invention, the heart side magnetic force generator is attached to the cover member which is to cover the heart, and therefore, the heart side magnetic force generator can be fixedly secured to the heart of the patient in a minimally invasive manner.

According to the third invention, the heart side magnetic force generator includes a plurality of magnetic force generators. Therefore, a contraction force and an expansion force generated by a magnetic force can be dispersedly exerted at a plurality of positions of the heart, so that the load on the cardiac muscle can be reduced, and sufficient pumping function can be achieved.

According to the fourth invention, the heart side magnetic force generators are fixedly secured to the front and rear surfaces of the heart. Therefore, the contraction force and the expansion force can be exerted on the heart on both the front and rear sides, and the pumping function can be effectively improved.

According to the fifth invention, the molded member is divided into the front divisional part and the rear divisional part, and these divisional parts have binding portions at which they are joined together. Therefore, the manipulation of placing the molded member so as to cover the heart can be facilitated.

According to the sixth invention, the molded member has a space into which the heart is to be inserted with the heart apex foremost. Therefore, the manipulation of placing the molded member so as to cover the heart can be facilitated.

### Brief Description of Drawings

FIG. 1 shows a heart assist device of embodiment 1 which is before attachment to the heart.
FIG. 2 is a block diagram of a controller.
FIG. 3 shows a state of a heart inserted in a net member.
FIG. 4 is a cross-sectional view of a magnetically contracted heart taken along line A-A of FIG. 1.
FIG. 5 shows a magnetically expanded heart, which corresponds to FIG. 4.
FIG. 6 is a perspective view of a molded member which is a variation of embodiment 1.
FIG. 7 is a perspective view of a molded member of embodiment 2.
FIG. 8 is a cross-sectional view taken along line B-B of FIG. 6.
FIG. 9 shows a variation of embodiment 2, which corresponds to FIG. 7.
FIG. 10 is a perspective view showing heart side magnets and molded member side magnets in the variations of embodiments 1 and 2.
FIG. 11 is a side view showing the heart side magnets and the molded member side magnets in the variations of embodiments 1 and 2.
FIG. 12 is a side view showing the magnetic force lines around the heart side magnets and the molded member side magnets in the variations of embodiments 1 and 2.

### Description of Reference Characters

- 1: heart assist device
- 10: net member (cover member)
- 20a, 21a: N-pole portion (heart side magnetic force generator)
- 30, 70: molded member
- 40, 41: molded member side magnet (molded member side magnetic force generator)
- 50: controller (polarity switching unit)
- 71: front divisional part
- 71a: binding portion
- 72: rear divisional part
- 72a: binding portion

### Best Mode for Carrying Out the Invention

Hereinafter, embodiments of the present invention are described in detail with reference to the drawings. Note that the following description of the preferred embodiments is exemplary in nature and does not intend to limit the present invention or its applications and uses.

### (Embodiment 1)

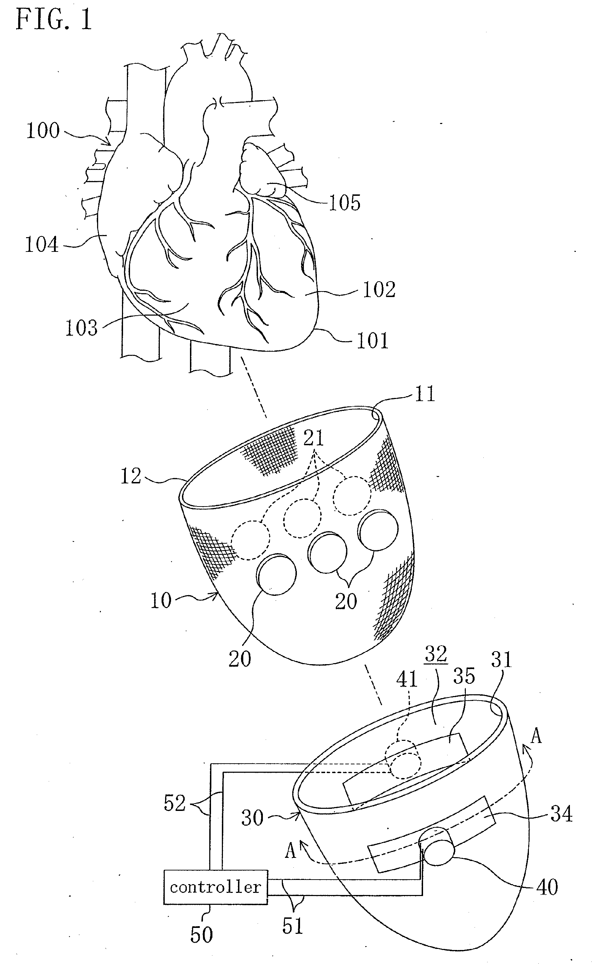

FIG. 1 shows a heart assist device 1 according to embodiment 1 of the present invention. The heart assist device 1 includes a net member 10 (cover member) for covering a heart 100 of a patient, a plurality of front and rear heart side magnets 20 and 21 attached to the net member 10, a molded member 30 which is shaped so as to cover the heart 100 with the net member 10 interposed therebetween, front and rear molded member side magnets 40 and 41 attached to the molded member 30, and a controller 50 (polarity switching unit).

The net member 10 has the shape of a bag which covers the surface of the heart 100 and is entirely constructed by weaving resin threads, or the like. The size of the net member 10 is larger than the heart 100 of the patient. The net member 10 has an opening 11 through which the heart 100 can be inserted with the heart apex 101 foremost. The heart 100 is inserted through the opening 11, and the opening 11 is closed by a string 12, whereby the net member 10 is attached to the heart 100 while covering the heart 100.

The front and rear heart side magnets 20 and 21 are formed by permanent magnets. The front heart side magnets 20 are three pieces of magnets which are provided in part of the net member 10 covering the front surface of the heart 100. The rear heart side magnets 21 are three pieces of magnets which are provided in part of the net member 10 covering the rear surface of the heart 100. The examples of the heart side magnets 20 and 21 include alnico magnets, ferrite magnets, rare-earth magnets, etc. Note that the front side of the heart 100 refers to the chest side of the patient, and the rear side refers to the back side.

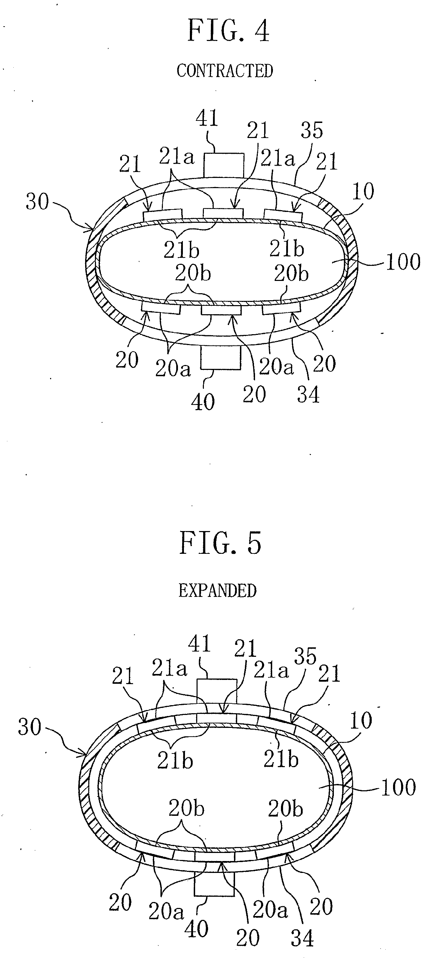

The front and rear heart side magnets 20 and 21 have the shape of a circular disk. One side of the respective magnets 20 and 21 with respect to the thickness direction constitutes N-pole portions 20a and 21a, and the other side constitutes S-pole portions 20b and 21b. The front and rear heart side magnets 20 and 21 are positioned so as to respectively correspond to the left ventricle 102 and the right ventricle 103 of the heart 100. As shown in FIG. 4, the magnets 20 and 21 are attached to the net member 10 using an adhesive, or the like, such that the N-pole portions 20a and 21a face outward of the net member 10, and the S-pole portions 20b and 21b face inward of the net member 10. Note that the heart side magnets 20 and 21 may be stitched onto the net member 10 using a thread. The heart side magnets 20 and 21 may be attached to any of the outer and inner surfaces of the net member 10. The N-pole portions 20a and 21a of the heart side magnets 20 and 21 constitute a heart side magnetic force generator of the present invention.

The molded member 30 is formed by molding a resin material which has a higher rigidity than the cardiac muscle of the heart 100 into the shape of a cup. The resin material is not limited to any particular type so long as it is highly biocompatible. The molded member 30 has an opening 31 through which the heart 100 can be inserted with the heart apex 101 foremost. The molded member 30 has an inner space 32 which is in communication with the opening 31. The size of the opening 31 is greater than the exterior of the heart 100. The depth of the space 32 of the molded member 30 is such a depth that the molded member 30 can cover the left ventricle 102, the right ventricle 103, and the right atrium 104 of the heart 100. A cross section of the molded member 30 taken along a direction perpendicular to the depth direction is greater than the exterior of the heart 100. Note that, in FIG. 1, character 105 indicates the left atrium.

Part of the molded member 30 corresponding to the front side of the heart 100 and the other part of the molded member 30 corresponding to the rear side respectively include a front side attaching portion 34 and a rear side attaching portion 35 to which the molded member side magnets 40 and 41 are to be attached. The attaching portions 34 and 35 are formed by molding a magnetic material. Specifically, the attaching portions 34 and 35 are insert-molded in the molding of the molded member 30 so as to be integral with the main part of the molded member 30. The magnetic material used for the attaching portions 34 and 35 may be iron, manganese, cobalt, nickel, etc., and is preferably a material without residual magnetism (e.g., soft iron).

The front side attaching portion 34 has the shape of a rectangular plate elongated in the direction of alignment of the three front heart side magnets 20. The shape of the front side attaching portion 34 is adapted such that the three front heart side magnets 20 are covered with the front side attaching portion 34 over the N-pole portions 20a when the heart 100 has been inserted in the molded member 30. The rear side attaching portion 35 also has the shape of a rectangular plate. The shape of the rear side attaching portion 35 is adapted such that the three rear heart side magnets 21 are covered with the rear side attaching portion 35 over the N-pole portions 21a when the heart 100 has been inserted in the molded member 30. The both surfaces of the front side and rear side attaching portions 34 and 35 are exposed to the inside and outside of the molded member 30.

The front and rear molded member side magnets 40 and 41 are formed by a coil of a copper wire, a core made of a magnetic material, and an electromagnet having a return yoke. The front and rear molded member side magnets 40 and 41 are positioned such that magnetic force lines which occur when an electric current is flowed are oriented in the directions between the inside and outside of the molded member 30, and are directly attached to the outer surfaces of the front side attaching portion 34 and the rear side attaching portion 35. Therefore, when an electric current is flowed through the coil of the front molded member side magnet 40 attached to the front side attaching portion 34, the front side attaching portion 34 is magnetized. When an electric current is flowed through the coil of the rear molded member side magnet 41 attached to the rear side attaching portion 35, the rear side attaching portion 35 is magnetized. The molded member side magnets 40 and 41 constitute a molded member side magnetic force generator of the present invention.

The controller 50 has connection lines 51 and 52 which are electrically coupled with the coils of the molded member side magnets 40 and 41, respectively. The controller 50 is configured to control the direction and magnitude of a direct current which is supplied to the coils via the connection lines 51 and 52. The controller 50 includes a signal generator 53 for generating a pulsatile signal, a current supply 54 for supplying a current to the molded member side magnets 40 and 41 based on the signal output from the signal generator 53, and a power supply 55.

The signal generator 53 has generally the same structure as those of cardiac pacemakers, or the like, conventionally used in heart disease patients. The pulsatile signal generated by the signal generator 53 has a waveform which includes, for example, 60 cycles of peak and valley within one minute. The number of peaks in the pulsatile signal can be set in the range of about 60-70. The signal generator 53 may have a heartbeat response function which is configured to automatically change the number of peaks in the pulsatile signal according to the activity conditions of the patient, etc.

The pulsatile signal generated by the signal generator 53 is input to the current supply 54. The current supply 54 is configured to change the direction of the current flowing through the coils at the peaks and valleys of the pulsatile signal. Specifically, when a current is supplied at the peaks of the pulsatile signal such that one side of the molded member side magnets 40 and 41 facing against the heart 100 serves as the N-pole portions while the other side of the magnets 40 and 41 opposite to the heart 100 serves as the S-pole portions, a current is supplied at the valleys of the pulsatile signal such that the side of the molded member side magnets 40 and 41 facing against the heart 100 serves as the S-pole portions while the other side of the magnets 40 and 41 opposite to the heart 100 serve as the N-pole portions. The current supply 54 is configured to concurrently supply currents of the same direction to both the front molded member side magnet 40 and the rear molded member side magnet 41. The magnitude of the currents supplied by the current supply 54 is determined such that a sufficient magnetic force is generated for contraction and pulsation of the heart 100 which will be described later. The value of the current supplied at the peaks of the pulsatile signal and the value of the current supplied at the valleys can be separately set to any values. One of the current values can be greater than the other. The timing at which the current is supplied to the front molded member side magnet 40 and the timing at which the current is supplied to the rear molded member side magnet 41 can be different.

The power supply 55 is formed by a battery, from which currents are supplied to the signal generator 53 and the current supply 54.

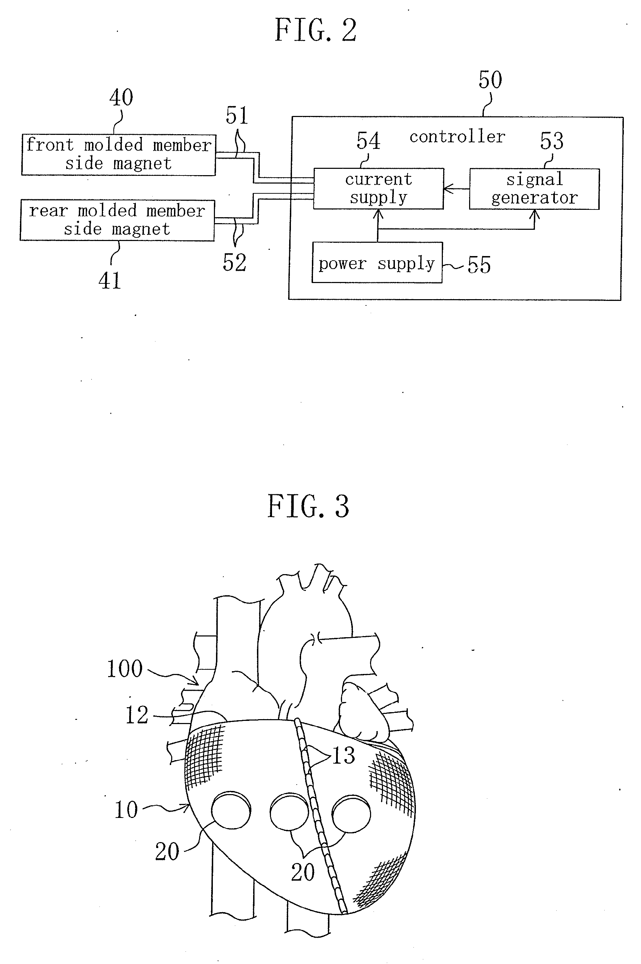

Next, attachment of the heart assist device 1 having the above-described structure to a patient is described. First, the chest of the patient is opened such that the heart 100 is exposed. Thereafter, the heart 100 is inserted into the net member 10 via the opening 11 with the heart apex 101 foremost. Here, the insertion can readily be carried out because the opening 11 is greater than the exterior of the heart 100. As shown in FIG. 3, generally the entirety of the left ventricle 102 and the right ventricle 103 of the heart 100 and the lower part of the right atrium 104 are inserted into the net member 10, and then, the opening 11 of the net member 10 is closed by the string 12. Also, part of the net member 10 is pinched to reduce the size of the net member 10 such that the entirety of the net member 10 comes in contact with the surface of the heart 100. In such a state, the pinched part of the net member 10 is stitched with a thread 13 to be bound. The heart 100 which has been covered with the net member 10 in this way is left for a while, so that the surface tissue of the heart 100 comes out through the meshes of the net member 10, and the heart 100 becomes integral with the net member 10. As a result, the net member 10 is in tight contact with and inseparable from the surface of the heart 100, and the front and rear heart side magnets 20 and 21 are fixedly secured to the heart 100. In the case of dilated cardiomyopathy, the size of the hypertrophied heart 100 can be decreased by decreasing the size of the net member 10.

Thereafter, the heart 100 is inserted with the heart apex 101 foremost into the molded member 30 via the opening 31. When the heart 100 is thoroughly inserted in the molded member 30, the front side attaching portion 34 and the front heart side magnets 20 face each other, and the rear side attaching portion 35 and the rear heart side magnets 21 also face each other. The controller 50 is buried in the body, as are the conventional cardiac pacemakers, with the connection lines 51 and 52 being coupled with the front and rear molded member side magnets 40 and 41.

Next, the operation of the heart assist device 1 is described. When a current is supplied from the current supply 54 of the controller 50 via the connection lines 51 to the coil of the molded member side magnet 40 positioned on the front side of the heart 100 such that the side of the molded member side magnet 40 facing against the heart 100 serves as the N-pole portion, the front side attaching portion 34 of the molded member 30 is magnetized. Meanwhile, a current is supplied from the current supply 54 via the connection lines 52 to the coil of the molded member side magnet 41 positioned on the rear side of the heart 100 so that the side of the molded member side magnet 41 facing against the heart 100 serves as the N-pole portion, whereby the rear side attaching portion 35 is magnetized.

Accordingly, a repulsive force occurs between the front molded member side magnet 40 and the front heart side magnets 20, and a repulsive force also occurs between the rear molded member side magnet 41 and the rear heart side magnets 21. Here, the molded member side magnets 40 and 41 hardly move relative to the heart 100 because the molded member 30 has a higher rigidity than the cardiac muscle and is shaped so as to cover the heart 100. Therefore, the heart side magnets 20 and 21 move in directions away from the molded member side magnets 40 and 41, respectively, and accordingly, the heart side magnets 20 and 21 are pressed against the heart 100. The heart 100, against which the heart side magnets 20 and 21 are pressed, contracts.

When the direction of the current supplied from the current supply 54 of the controller 50 is changed, the side of the front molded member side magnet 40 facing against the heart 100 becomes the S-pole portion, and the front side attaching portion 34 is magnetized. Meanwhile, the side of the rear molded member side magnet 41 facing against the heart 100 becomes the S-pole portion, and the rear side attaching portion 35 is magnetized. Thus, the front and rear heart side magnets 20 and 21 are attracted by the front and rear molded member side magnets 40 and 41, respectively. Here, since the front and rear heart side magnets 20 and 21 are attached to the net member 10 integrated with the heart 100, the cardiac muscle is pulled together with the net member 10 when the heart side magnets 20 and 21 are attracted by the molded member side magnets 40 and 41. Accordingly, the heart 100 expands.

As described above, a contraction force and an expansion force can be repeatedly applied to the heart 100 of the patient so that the heart 100 of the patient can be caused to pulsate. Here, for example, a greater contraction force and a greater expansion force can be applied to the left ventricle 102 by densely providing the heart side magnets 20 and 21, or using larger magnets, in part of the heart 100 corresponding to the left ventricle 102.

As described above, in the heart assist device 1 of this embodiment, the heart side magnets 20 and 21 are fixedly secured to the heart 100, and the polarity of the molded member side magnets 40 and 41 provided to the molded member 30 covering the heart 100 is switched by the controller 50 at predetermined timings. Therefore, a contraction force and an expansion force can be magnetically applied to the heart 100 of the patient so that the heart 100 pulsates without using a conventional mechanical pump, and the pumping function can be improved. Since the heart of the patient can be caused to pulsate in this way, air does not enter the blood, and formation of a thrombus rarely occurs. Thus, the burden on the patient can be reduced.

Since the heart side magnets 20 and 21 are attached to the net member 10 which covers the surface of the heart 100, the heart side magnets 20 and 21 can be fixedly secured to the heart 100 of the patient in a minimally invasive manner without substantial damage.

Since the heart assist device 1 includes a plurality of pieces of the heart side magnets 20 and a plurality of pieces of the heart side magnets 21, a contraction force and an expansion force generated by a magnetic force can be dispersedly exerted on a plurality of positions of the heart 100. Thus, the load on the cardiac muscle can be decreased, and a sufficient pumping function can be obtained.

Since the heart side magnets 20 and 21 are fixedly secured respectively to the front and rear surfaces of the heart 100, a contraction force and an expansion force can be exerted on the heart 100 on both the front and rear sides. Thus, the pumping function can be effectively improved.

Since the molded member 30 has the space 32 into which the heart 100 is to be inserted with the heart apex 101 foremost, the manipulation of placing the molded member 30 so as to cover the heart 100 can be facilitated.

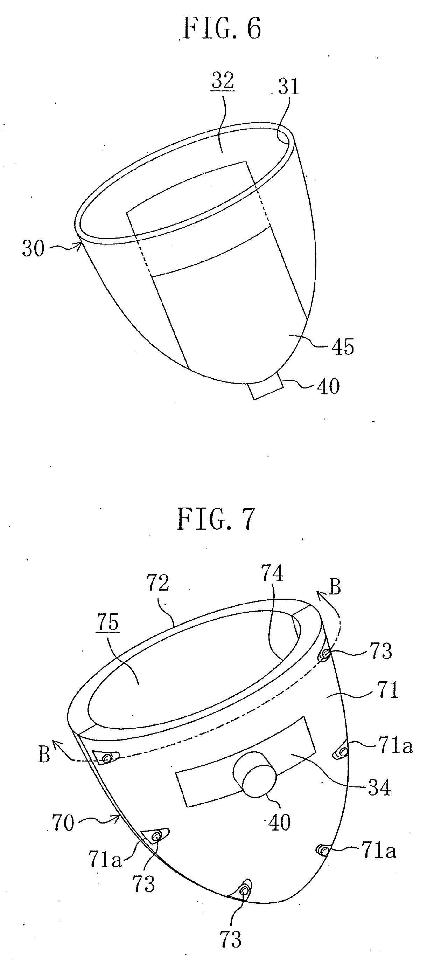

As in a variation of embodiment 1 which is shown in FIG. 6, a single piece of magnet 40 may be fixed to a single attaching portion 45. The attaching portion 45 is made of the above-described magnetic material. The attaching portion 45 extends from part of the molded member 30 corresponding to the heart apex toward the opening 31 such that the attaching portion 45 can sandwich the heart 100 in the anteroposterior direction. The magnet 40 is attached to part of the attaching portion 45 corresponding to the heart apex. The magnetic force of the magnet 40 acts on the entirety of the attaching portion 45. Thus, in this variation, the pumping function can be improved by applying a magnetic force to the heart 100 on both sides of the heart 100 in the anteroposterior direction with only a single piece of the magnet 40 used.

### (Embodiment 2)

FIG. 7 shows a molded member 70 of a heart assist device according to embodiment 2 of the present invention. The heart assist device of embodiment 2 is different from that of embodiment 1 only in the structure of the molded member 70, and the other elements are the same. Therefore, hereinafter, the same elements as those of embodiment 1 are denoted by the same reference characters and the description thereof is omitted. The differences are described in detail below.

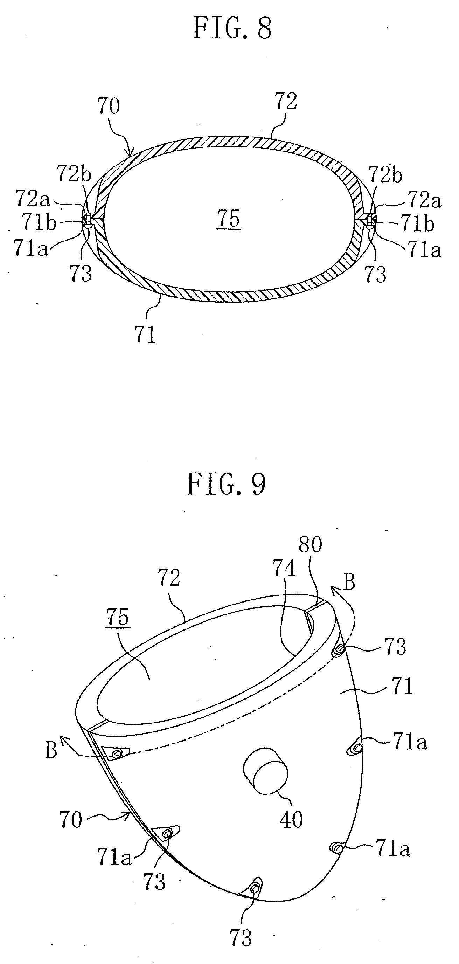

The molded member 70 of embodiment 2 is composed of two divisional parts having the shape of halved elements, a front divisional part 71 and a rear divisional part 72, which are separate with respect to the anteroposterior direction of the heart 100. As shown in FIG. 8, the front divisional part 71 and the rear divisional part 72 are integrally joined by screws 73. When joined, the front divisional part 71 and the rear divisional part 72 define an opening 74 and a space 75. Part of the front divisional part 71 which is to be bound to the rear divisional part 72 has front screwing portions 71a (binding portions). The front screwing portions 71a have screw insertion holes 71b. Part of the rear divisional part 72 which is to be bound to the front divisional part 71 has rear screwing portions 72a (binding portions). The rear screwing portions 72a have internally threaded holes 72b into which the screws 73 are to be screwed.

In the process of attaching the molded member 70 to the heart 100, the front divisional part 71 and the rear divisional part 72 are first separate from each other. The heart 100 is placed between the divisional parts 71 and 72, and then, the screws 73 are inserted through the screw insertion holes 71b and screwed into the internally threaded holes 72b, whereby the divisional parts 71 and 72 are integrally joined together.

The heart assist device of embodiment 2 also enjoys substantially the same advantages as does the device of embodiment 1. Since in embodiment 2 the molded member 70 is composed of two divisional parts 71 and 72, the manipulation of placing the molded member 70 so as to cover the heart 100 can be facilitated.

When joining the front divisional part 71 and the rear divisional part 72 together, a binding tool other than screws, such as rivets or the like, may be used.

As shown in FIG. 9, the front divisional part 71 and the rear divisional part 72 may be made of the above-described magnetic material. In this case, the molded member side magnets 40 and 41 are directly attached to the front divisional part 71 and the rear divisional part 72, respectively. Between the front divisional part 71 and the rear divisional part 72, for example, a resin member 80 may be interposed, such that the front divisional part 71 and the rear divisional part 72 are not in contact with each other, for preventing transmission of the magnetic force of the front molded member side magnet 40 to the rear divisional part 72.

In embodiment 2, an elastic member may be interposed between the front divisional part 71 and the rear divisional part 72.

Note that the heart side magnets may be fixedly secured to only one of the front and rear surfaces of the heart 100 although in embodiments 1 and 2 the heart side magnets 20 and 21 are fixedly secured to the front and rear surfaces of the heart 100. In this case, one of the molded member side magnets 40 and 41 may be omitted. The number of the heart side magnets 20 and 21 is not limited to three, but may be one or may be four or more. The shape of the heart side magnets 20 and 21 is not limited to the shape of a circular disk, but may be the shape of a rectangular plate.

The molded members 30 and 70 may be made of a material other than resin.

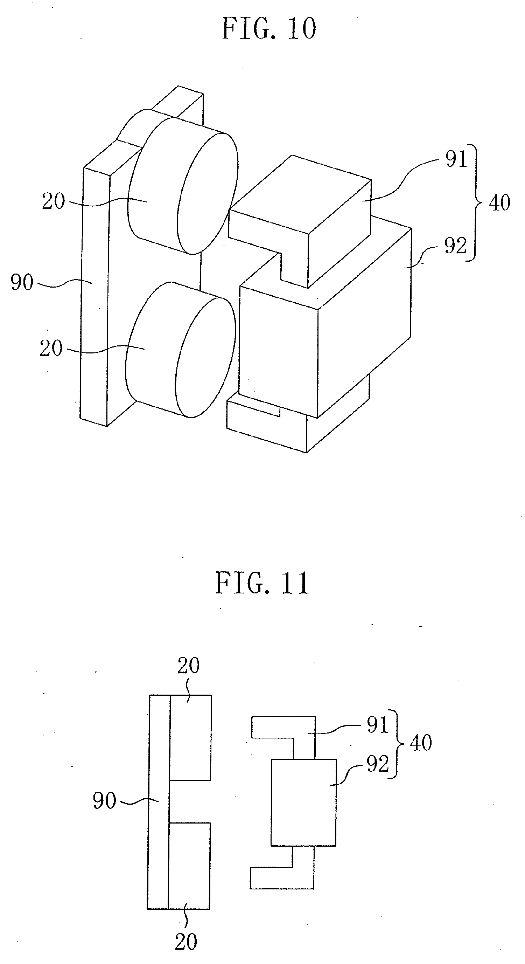

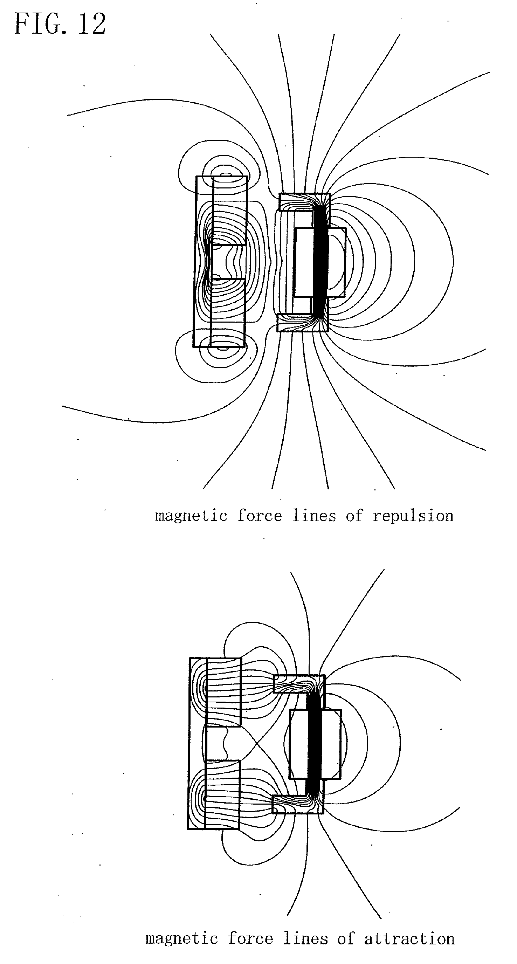

As shown in FIG. 10 and FIG. 11, two pieces of the heart side magnets 20 may be fixed to a back yoke 90, and the molded member side magnet 40 may be provided with an electromagnet side yoke 91. In FIG. 10 and FIG. 11, character 92 indicates a coil. Among the two heart side magnets 20, the S-pole portion of one heart side magnet 20 faces against one end of the electromagnet side yoke 91 of the molded member side magnet 40, and the N-pole portion of the other heart side magnet 20 faces against the other end of the electromagnet side yoke 91 of the molded member side magnet 40. When an electric current is flowed through the coil 92 of the molded member side magnet 40, magnetic force lines are generated as shown in FIG. 12. A repulsive force or attractive force is obtained between the heart side magnets 20 and the molded member side magnet 40 according to the direction of the current. The heart side magnets 21 and the molded member side magnet 41 can also be configured as illustrated in this variation.

### Industrial Applicability

As described above, a heart assist device of the present invention is suitable for, for example, patients suffering from diastolic failure and systolic failure in a ventricle, such as dilated cardiomyopathy patients.

## Claims

1. A heart assist device, comprising:
a heart side magnetic force generator fixedly secured to a surface of a heart;
a molded member formed of a material having a higher rigidity than a cardiac muscle so as to cover the heart;
a molded member side magnetic force generator provided to the molded member so as to correspond to the heart side magnetic force generator; and
a polarity switching unit configured to switch a polarity of one of the heart side magnetic force generator and the molded member side magnetic force generator at predetermined timings.

2. The heart assist device of claim 1, further comprising a cover member configured to cover the surface of the heart, wherein
the heart side magnetic force generator is attached to the cover member.

3. The heart assist device of claim 1, wherein the heart side magnetic force generator includes a plurality of magnetic force generators.

4. The heart assist device of claim 3, wherein the heart side magnetic force generators are fixedly secured to a front surface and a rear surface of the heart.

5. The heart assist device of claim 1, wherein
the molded member is composed of a front divisional part and a rear divisional part which are separate with respect to an anteroposterior direction of the heart, and
the front divisional part and the rear divisional part each have a binding portion at which the front divisional part and the rear divisional part are joined together.

6. The heart assist device of claim 1, wherein the molded member has a space into which the heart is to be inserted with a heart apex foremost.
